# EUROPEAN PATENT APPLICATION

(11) **EP 3 260 444 A1**
(43) Date of publication of application: **27.12.2017**
(21) Application number: 15882518.2
(22) Date of filing: 14.12.2015
(51) Int. Cl.: C07C 381/12, C07F 5/02, C08G 59/68, C08K 5/55, C08L 101/00, C09K 3/00

(54) **SULFONIUM BORATE SALT, ACID GENERATING AGENT AND CURABLE COMPOSITION**

(30) Priority: 16.02.2015 JP 2015027294
(71) Applicant: San-Apro Ltd., Kyoto-shi, Kyoto 605-0995 (JP)
(72) Inventor: IKEDA, Takuya, Kyoto-shi, Kyoto 6050995 (JP)
(74) Representative: Maiwald Patentanwalts GmbH
(86) International application number: PCT/JP2015/006216
(87) International publication number: WO 2016/132413

(57) **Abstract**

Provided is a sulfonium borate salt that is free from highly toxic elements such as Sb, has a high sensitivity to active energy rays such as heat or light, and shows an excellent storage stability when blended with a cationic polymerizable compound such as an epoxy compound. The sulfonium borate salt according to the present invention that is represented by general formula (1); and an acid generating agent, comprising the sulfonium borate salt. In general formula (1): R¹ represents an alkyl group; and R² represents a benzyl group represented by general formula (2).

## Description

### TECHNICAL FIELD

The present invention firstly relates to a sulfonium borate salt, and secondly relates to an acid generating agent, more particularly, to an acid generating agent containing a specific sulfonium borate salt which is suitable when heat or an active energy ray such as light, an electron ray, or an X-ray is made to act on the acid generating agent to cure a cationic polymerizable compound. The present invention thirdly relates to a curable composition containing the acid generating agent and a cured product obtained by curing the composition.

### BACKGROUND ART

As a cationic polymerization initiator on which heat or an active energy ray such as light, an electron ray, or an X-ray is made to act to cure a cationic polymerizable compound such as an epoxy compound, a sulfonium salt has hitherto been known.

Incidentally, although cationic polymerization initiators described in these specifications contain BF₄⁻, PF₆⁻, AsF₆⁻, or SbF₆⁻ as an anion, initiation performance for cationic polymerization varies with the kind of an anion and the respective initiators are improved in an ascending order of BF₄⁻< PF₆⁻ < AsF₆⁻ < SbF₆⁻. However, applications of cationic polymerization initiators containing AsF₆⁻ or SbF₆⁻ being satisfactory in the polymerization-initiating ability are limited from a toxicity problem of As or Sb, and only an SbF₆⁻salt is used in a limited field such as photofabrication. On that account, although a PF₆⁻ salt being poor in the polymerization-initiating ability is generally utilized, for example, in order to attain a curing rate almost comparable to that of an SbF₆⁻ salt, since the PF₆⁻ salt in an amount being nearly ten times the amount of the SbF₆⁻ salt needs to be added and the remaining amount of an unreacted initiator, a solvent used as necessary for dissolving the initiator, or a decomposition product of the initiator is increased, there are problems that physical properties of a cured material are impaired, and moreover, a base material, facilities, and the like are liable to be corroded because of an increased amount of HF produced as a by-product by decomposition of the initiator. As such, a cationic polymerization initiator that is free from a toxic metal and has a cationic polymerization-initiating ability comparable to that of an SbF₆⁻ salt has been strongly required. As such, in recent years, a sulfonium borate salt having a counter anion in which a fluorine atom bonds to a carbon atom and which has a boron atom, instead of SbF₆⁻, has been used as a cationic polymerization initiator (Patent Documents 5, 6, 7 and 8) .

### PRIOR ART DOCUMENTS

### PATENT DOCUMENTS

Patent Document 1: JP-A-02-196812
Patent Document 2: JP-A-03-17101
Patent Document 3: JP-A-03-237107
Patent Document 4: JP-A-03-205405
Patent Document 5: JP-A-2008-303167
Patent Document 6: JP-A-2010-132614
Patent Document 7: JP-A-2012-153642
Patent Document 8: JP-A-2010-201611

### DISCLOSURE OF THE INVENTION

### PROBLEMS TO BE SOLVED BY THE INVENTION

The sulfonium borate salt described in Patent Documents 5 to 8 is excellent in activity against heat, but poor in stability at room temperature, and has a drawback that, when blended with a cationic polymerizable compound, the pot life of a blend is short.

Accordingly, firstly, an object of the present invention is to provide a new sulfonium salt that is free from highly toxic elements such as Sb, has an excellent cationically polymerizing ability, and shows excellent preservation stability when blended with a cationic polymerizable compound.

Secondly, an object of the present invention is to provide a new acid generating agent including a sulfonium borate salt that is free from highly toxic elements such as Sb, has an excellent cationically polymerizing ability, and shows excellent preservation stability when blended with a cationic polymerizable compound.

Thirdly, an object of the present invention is to provide a curable composition in which the above-described acid generating agent is utilized and a cured product thereof.

### SOLUTIONS TO THE PROBLEMS

The present inventor has found that a sulfonium borate salt represented by the following formula (1) is suitable for the above-described objects. That is, the present invention provides a sulfonium borate salt represented by formula (1).

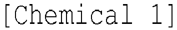

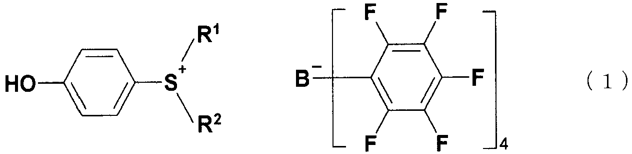

[In formula (1), R¹ represents an alkyl group, and R² represents a benzyl group represented by the following general formula (2).]

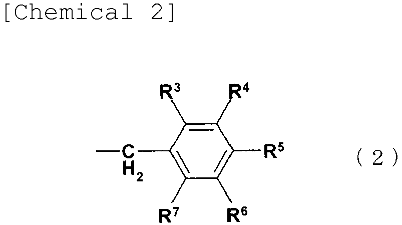

[In formula (2), R³ to R⁷ independently represent a hydrogen atom or an electron-attracting group, and the sum of Hammett substituent constant σ values of R³ to R⁷ is 0.43 or more.]

Moreover, the present invention is directed to an acid generating agent comprising the above-described sulfonium borate salt.

Moreover, the present invention is directed to a curable composition including the above-described acid generating agent and a cationic polymerizable compound.

Furthermore, the present invention is directed to a cured product obtained by curing the above-described curable composition.

### EFFECTS OF THE INVENTION

The sulfonium borate salt of the present invention is free from highly toxic elements such as Sb, and shows excellent preservation stability when blended with a cationic polymerizable compound.

### MODE FOR CARRYING OUT THE INVENTION

Hereinafter, the embodiment according to the present invention will be described in detail.

In formula (1), examples of an alkyl group as R¹ include straight-chain alkyl groups having 1 to 18 carbon atoms (methyl, ethyl, n-propyl, n-butyl, n-pentyl, n-octyl, n-decyl, n-dodecyl, n-tetradecyl, n-hexadecyl, n-octadecyl, and the like), branched-chain alkyl groups having 1 to 18 carbon atoms (isopropyl, isobutyl, sec-butyl, tert-butyl, isopentyl, neopentyl, tert-pentyl, isohexyl, and isooctadecyl), cycloalkyl groups having 3 to 18 carbon atoms (cyclopropyl, cyclobutyl, cyclopentyl, cyclohexyl, 4-decylcyclohexyl, and the like), and the like.

Among these, from the viewpoint of reactivity, preferred are straight-chain alkyl groups having 1 to 4 carbon atoms, and especially preferred is a methyl group.

In formula (1), R² represents a benzyl group represented by the foregoing general formula (2). In formula (2), R³ to R⁷ independently represent a hydrogen atom or an electron-withdrawing group, and the sum of Hammett substituent constant σ values of R³ to R⁷ is 0.43 or more.

In the present invention, an electron-attracting group as each of R³ to R⁷ refers to a substituent with a Hammett substituent constant σ value of a positive value. For example, the σ value of Hammett is described in detail in general remarks by Yuho Tsuno (Journal of synthetic organic chemistry, Vol. 23, No. 8, (1965), p. 631-642), "Cram ORGANIC CHEMISTRY [II] 4th Edition" translated by Yasuhide Yukawa, p. 656, (Hirokawa-Shoten Ltd.), and the like. In this connection, the electron-attracting group is prescribed by the σ value in the present invention, but substituents are not limited to those described in the above-described documents having known values.

Examples of the electron-attracting group with an σ value of a positive value include a m-alkoxy group, a m-hydroxy group, a halogen atom, a halogenated alkyl group, an acyloxy group, an acyl group, a cyano group, a nitro group, and a sulfonyl group. Substituents represented by R³ to R⁷ previously described may be selected so that the sum of Hammett substituent constant σ values of R³ to R⁷ becomes 0.43 or more.

From the viewpoint of storage stability, the sum of Hammett substituent constant σ values of R³ to R⁷ is preferably 0.70 or more. The upper limit thereof is usually 3.00 or less, and preferably 2.00 or less.

Examples of the m-alkoxy group as the above-described electron-attracting group include m-methoxy, m-ethoxy, m-normalpropoxy, m-isopropoxy, m-normalbutoxy, m-isobutoxy, m-tert-butoxy, and the like.

Examples of the halogen atom include a fluorine atom, a chlorine atom, a bromine atom, an iodine atom, and the like.

Examples of the halogenated alkyl group include a perfluoroalkyl group in which some or all of hydrogen atoms in an alkyl group are substituted by fluorine atoms, and examples of the alkyl group include straight-chain alkyl groups (methyl, ethyl, propyl, butyl, pentyl, octyl, and the like), branched-chain alkyl groups (isopropyl, isobutyl, sec-butyl, tert-butyl, and the like), cycloalkyl groups (cyclopropyl, cyclobutyl, cyclopentyl, cyclohexyl, and the like), and the like.

Examples of the acyloxy group include acetoxy, butanoyloxy, benzoyloxy, and the like.

Examples of the acyl group include acetyl, ethanoyl, propanoyl, butanoyl, pivaloyl, benzoyl, and the like.

Examples of the sulfonyl group include methanesulfonyl, benzenesulfonyl, toluenesulfonyl, trifluoromethanesulfonyl, difluoromethanesulfonyl, and the like.

Among examples of R², preferred are a p-nitrobenzyl group, a m-nitrobenzyl group, a p-cyanobenzyl group, a m-cyanobenzyl group, a p-trifluoromethylbenzyl group, a m-trifluoromethylbenzyl group, a pentafluorobenzyl group, a 3,5-bis(trifluoromethyl)benzyl group, a 2,4-bis(trifluoromethyl)benzyl group, and a 2,5-bis(trifluoromethyl)benzyl group.

With regard to a production method of a sulfonium borate salt of the present invention, the sulfonium borate salt can be produced according to the following reaction equations.

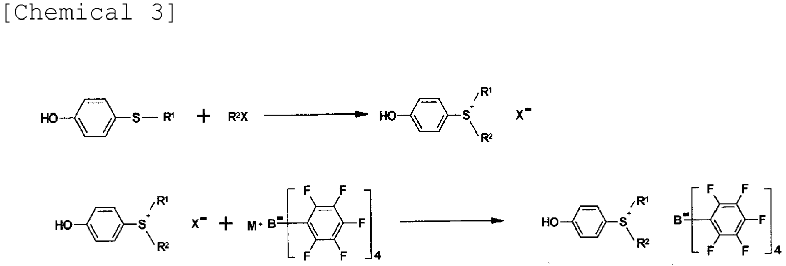

In this connection, the same definitions as those in formula (1) hold for R¹ and R² in the reaction equations. X represents a halogen atom, and M⁺ represents an alkali metal (lithium, sodium, potassium, or the like) cation. MX represents a salt between an alkali metal cation and a halogen anion.

In the above-described reaction equations, a reaction at the first stage may be performed under a solventless condition, or may be performed in water, an organic solvent, or a mixture thereof, as necessary. Although the reaction temperature varies depending on the boiling point of a solvent used, the reaction temperature is -20 to 80°C or so. The reaction time is 1 to several tens of hours or so.

A reaction at the second stage may be performed subsequently to the reaction at the first stage, and a sulfonium halide may be previously isolated (and purified as necessary) to perform a reaction at the second stage. A sulfonium halide and an aqueous solution of a salt between an alkali metal cation and a tetrakis (pentafluorophenyl)borate are mixed and stirred to perform a metathesis reaction, and by filtering off a solid obtained as a precipitate or extracting an oily substance separated with an organic solvent and then removing the organic solvent, a sulfonium borate salt of the present invention is obtained as a solid or a viscous liquid. The resulting solid or viscous liquid can be washed with a suitable organic solvent or purified by a recrystallization method or a column chromatography method as necessary.

The chemical structure of a sulfonium borate salt of the present invention can be identified by a general analytical procedure (for example, ¹H-, ¹¹B-, ¹³C-, ¹⁹F-, or ³¹P-nuclear magnetic resonance spectrum, infrared absorption spectrum, and/or elemental analysis or the like).

A sulfonium borate salt of the present invention is suitable as an acid generating agent. The acid generating agent refers to an agent allowing its chemical structure to be decomposed by heating or photoirradiation and generating an acid. The acid generated can be used as a catalyst for a curing reaction of an epoxide or the like.

A sulfonium borate salt of the present invention may be singly used as an acid generating agent of the present invention, and the acid generating agent may be made to contain another acid generating agent to be used.

In the case where another acid generating agent is contained, the content (% by mole) of another acid generating agent is preferably 1 to 100 and further preferably 5 to 50 relative to the total number of moles of a sulfonium borate salt of the present invention.

As examples of another acid generating agent, conventionally known agents such as an onium salt (sulfonium, iodonium, selenium, ammonium, phosphonium, or the like) and a salt between a transition metal complex ion and an anion are included therein.

An acid generating agent of the present invention may be previously dissolved in a solvent, which does not impair the cationic polymerization, in order to facilitate the compatibilization thereof with a cationic polymerizable compound.

Examples of the solvent include carbonates such as propylene carbonate, ethylene carbonate, 1,2-butylene carbonate, dimethyl carbonate, and diethyl carbonate; ketones such as acetone, methyl ethyl ketone, cyclohexanone, methyl isoamyl ketone, and 2-heptanone; polyhydric alcohols such as ethylene glycol, ethylene glycol monoacetate, diethylene glycol, diethylene glycol monoacetate, propylene glycol, propylene glycol monoacetate, dipropylene glycol, a monomethyl ether, monoethyl ether, monopropyl ether, monobutyl ether, or monophenyl ether of dipropylene glycol monoacetate, and derivatives thereof; cyclic ethers such as dioxane; esters such as ethyl formate, methyl lactate, ethyl lactate, methyl acetate, ethyl acetate, butyl acetate, methyl pyruvate, methyl acetoacetate, ethyl acetoacetate, ethyl pyruvate, ethyl ethoxyacetate, methyl methoxypropionate, ethyl ethoxypropionate, methyl 2-hydroxypropionate, ethyl 2-hydroxypropionate, ethyl 2-hydroxy-2-methylpropionate, methyl 2-hydroxy-3-methylbutanoate, 3-methoxybutyl acetate, and 3-methyl-3-methoxybutyl acetate; aromatic hydrocarbons such as toluene and xylene, and the like.

In the case of using a solvent, the proportion of the solvent used is preferably 15 to 1000 parts by weight and further preferably 30 to 500 parts by weight relative to 100 parts by weight of the acid generating agent of the present invention. With regard to the solvent used, one kind thereof may be used alone and two or more kinds thereof may be used in combination.

A curable composition of the present invention is constituted of the above-described acid generating agent and a cationic polymerizable compound.

Examples of the cationic polymerizable compound include annular ethers (epoxides, oxetanes, and the like), ethylenically unsaturated compounds (vinyl ethers, styrene, and the like), bicyclo orthoesters, spiro orthocarbonates, spiro orthoesters, and the like (JP-A-11-060996, JP-A-09-302269, JP-A-2003-026993, JP-A-2002-206017, JP-A-11-349895, JP-A-10-212343, JP-A-2000-119306, JP-A-10-67812, JP-A-2000-186071, JP-A-08-85775, JP-A-08-134405, JP-A-2008-20838, JP-A-2008-20839, JP-A-2008-20841, JP-A-2008-26660, JP-A-2008-26644, JP-A-2007-277327, "Photopolymer Handbook" edited by Technical Association of Photopolymers, (1989, published by Kogyo Chosakai Publishing Co., Ltd.), "Technology of UV/EB Curing" edited by United Engineering Center, (1982, published by United Engineering Center), "UV/EB Curable Materials" edited by RadTech Japan, (1992, published by CMC Publishing Co., Ltd.), "UV koka ni okeru koka furyo•sogai genin to sono taisaku" edited by TECHNICAL INFORMATION INSTITUTE CO., LTD., (2003, published by TECHNICAL INFORMATION INSTITUTE CO., LTD.), Journal of the Japan Society of Color Material, Vol. 68, (5), p. 286-293, (1995), Fine Chemical, Vol. 29, (19), p. 5-14, (2000), and the like).

As the epoxide, a known one and the like can be used, and aromatic epoxides, alicyclic epoxides, and aliphatic epoxides are included therein.

Examples of the aromatic epoxide include glycidyl ethers of monovalent or polyvalent phenols having at least one aromatic ring (phenol, biphenols, bisphenol A, bisphenol F, phenol novolak, cresol novolak, and bromides thereof or alkylene oxide-adduct compounds by addition reaction of an alkylene oxide therewith) and glycidyl esters (glycidyl phthalate, glycidyl-3-methyl phthalate, and the like) of monovalent or polyvalent carboxylic acids having at least one aromatic ring (phthalic acid, 3-methylphthalic acid, and the like).

Examples of the alicyclic epoxide include a compound obtained by epoxidizing a compound having at least one cyclohexene or cyclopentene ring with an oxidizing agent (3,4-epoxycyclohexylmethyl-3,4-epoxycyclohexanecarboxylate or the like).

Examples of the aliphatic epoxide include polyglycidyl ethers of aliphatic polyhydric alcohols or alkylene oxide-adducts thereof (1,4-butanediol diglycidyl ether, 1,6-hexanediol diglycidyl ether, and the like), polyglycidyl esters of aliphatic polybasic acids (diglycidyl tetrahydrophthalate and the like), epoxidized products of long-chain unsaturated compounds (epoxidized soybean oil, epoxidized polybutadienes, and the like).

As the oxetane, a known one and the like can be used, and examples thereof include 3-ethyl-3-hydroxymethyloxetane, 2-ethylhexyl(3-ethyl-3-oxetanylmethyl)ether, 2-hydroxyethyl(3-ethyl-3-oxetanylmethyl)ether, 2-hydroxypropyl(3-ethyl-3-oxetanylmethyl)ether, 1,4-bis[(3-ethyl-3-oxetanylmethoxy)methyl]benzene, oxetanylsilsesquioxetane, phenol novolak oxetane, and the like.

As the ethylenically unsaturated compound, a known cationic polymerizable monomer and the like can be used, and aliphatic monovinyl ethers, aromatic monovinyl ethers, polyfunctional vinyl ethers, styrene, and cationic polymerizable nitrogen-containing monomers are included therein.

Examples of the aliphatic monovinyl ether include methyl vinyl ether, ethyl vinyl ether, butyl vinyl ether, cyclohexyl vinyl ether, and the like.

Examples of the aromatic monovinyl ether include 2-phenoxyethyl vinyl ether, phenyl vinyl ether, p-methoxyphenyl vinyl ether, and the like.

Examples of the polyfunctional vinyl ether include butanediol-1,4-divinyl ether, triethylene glycol divinyl ether, and the like.

Examples of respective kinds of styrene include styrene, α-methylstyrene, p-methoxystyrene, p-tert-butoxystyrene, and the like.

Examples of the cationic polymerizable nitrogen-containing monomer include N-vinylcarbazole, N-vinylpyrrolidone, and the like.

Examples of the bicyclo orthoester include 1-phenyl-4-ethyl-2,6,7-trioxabicyclo[2.2.2]octane, 1-ethyl-4-hydroxymethyl-2,6,7-trioxabicyclo-[2.2.2]octane, and the like.

Examples of the spiro orthocarbonate include 1,5,7,11-tetraoxaspiro[5.5]undecane, 3,9-dibenzyl-1,5,7,11-tetraoxaspiro[5.5]undecane, and the like.

Examples of the spiro orthoester include 1,4,6-trioxaspiro[4.4]nonane, 2-methyl-1,4,6-trioxaspiro[4.4]nonane, 1,4,6-trioxaspiro[4.5]decane, and the like.

Among these cationic polymerizable compounds, epoxides, oxetanes, and vinyl ethers are preferred, further preferred are epoxides and oxetanes, and especially preferred are alicyclic epoxides and oxetanes. Moreover, these cationic polymerizable compounds may be used alone and two or more kinds thereof may be used in combination.

The content of the acid generating agent of the present invention in a curable composition is preferably 0.05 to 20 parts by weight and further preferably 0.1 to 10 parts by weight relative to 100 parts of the cationic polymerizable compound. When the content lies within this range, a polymerization degree of the cationic polymerizable compound is further sufficiently increased and physical properties of a cured product become further satisfactory. In this connection, various factors such as the property of a cationic polymerizable compound, the kind of an energy ray and the irradiation amount thereof, the temperature, the curing time, the humidity, and the thickness of a coating film are taken into consideration in deciding this content, and the content is not limited within the above-described range.

The curable composition of the present invention can be made to contain known additives (a sensitizer, a pigment, a kind of filler, an antistatic agent, a flame retardant, a defoaming agent, a fluidity regulating agent, a light stabilizer, an oxidation inhibitor, an adhesion imparting agent, an ion scavenger, a coloration inhibitor, a solvent, a non-reactive resin, a radical polymerizable compound, and the like), as necessary.

Basically, a sensitizer does not need to be contained in the curable composition of the present invention, but a sensitizer as an agent that complements the curability can be contained therein, as necessary. As such a sensitizer, a known (JP-A-11-279212, JP-A-09-183960, or the like) sensitizer and the like can be used, and examples thereof include respective kinds of anthracene {anthracene, 9,10-dibutoxyanthracene, 9,10-dimethoxyanthracene, 9,10-diethoxyanthracene, 2-ethyl-9,10-dimethoxyanthracene, 9,10-dipropoxyanthracene, and the like}; pyrene; 1,2-benzanthracene; perylene; tetracene; coronene; respective kinds of thioxanthone {thioxanthone, 2-methylthioxanthone, 2-ethylthioxanthone, 2-chlorothioxanthone, 2-isopropylthioxanthone, 2,4-diethylthioxanthone, and the like}; respective kinds of phenothiazine {phenothiazine, N-methylphenothiazine, N-ethylphenothiazine, N-phenylphenothiazine, and the like}; xanthone; naphthalene and derivatives thereof {1-naphthol, 2-naphthol, 1-methoxynaphthalene, 2-methoxynaphthalene, 1,4-dihydroxynaphthalene, 4-methoxy-1-naphthol, and the like}; ketones {dimethoxyacetophenone, diethoxyacetophenone, 2-hydroxy-2-methyl-1-phenylpropane-1-one, 4'-isopropyl-2-hydroxy-2-methylpropiophenone, 4-benzoyl-4'-methyldiphenyl sulfide, and the like}; carbazole and derivatives thereof {N-phenylcarbazole, N-ethylcarbazole, poly-N-vinylcarbazole, N-glycidylcarbazole, and the like}; chrysene and derivatives thereof {1,4-dimethoxychrysene, 1,4-di-α-methylbenzyloxychrysene, and the like}; phenanthrene and derivatives thereof {9-hydroxyphenanthrene, 9-methoxyphenanthrene, 9-hydroxy-10-methoxyphenanthrene, 9-hydroxy-10-ethoxyphenanthrene, and the like}, and the like.

In the case where the curable composition contains a sensitizer, the content of the sensitizer is preferably 1 to 300 parts by weight and further preferably 5 to 200 parts by weight relative to 100 parts of the acid generating agent.

As the pigment, a known pigment and the like can be used, and examples thereof include inorganic pigments (titanium oxide, iron oxide, carbon black, and the like), organic pigments (azo pigments, cyanine pigments, phthalocyanine pigments, quinacridone pigments, and the like), and the like.

In the case where the curable composition contains a pigment, the content of the pigment is preferably 0.5 to 400000 parts by weight and further preferably 10 to 150000 parts by weight relative to 100 parts of the acid generating agent.

As the filler, known filler and the like can be used, and examples thereof include fused silica, crystalline silica, calcium carbonate, aluminum oxide, aluminum hydroxide, zirconium oxide, magnesium carbonate, mica, talc, calcium silicate, aluminum lithium silicate, and the like.

In the case where the curable composition contains a kind of filler, the content of the filler is preferably 50 to 600000 parts by weight and further preferably 300 to 200000 parts by weight relative to 100 parts of the acid generating agent.

As the antistatic agent, a known antistatic agent and the like can be used, and examples thereof include nonionic antistatic agents, anionic antistatic agents, cationinc antistatic agents, ampholytic antistatic agents, and polymeric antistatic agents.

In the case where the curable composition contains an antistatic agent, the content of the antistatic agent is preferably 0.1 to 20000 parts by weight and further preferably 0.6 to 5000 parts by weight relative to 100 parts of the acid generating agent.

As the flame retardant, a known flame retardant and the like can be used, and examples thereof include inorganic flame retardants {antimony trioxide, antimony pentoxide, tin oxide, tin hydroxide, molybdenum oxide, zinc borate, barium metaborate, red phosphorus, aluminum hydroxide, magnesium hydroxide, calcium aluminate, and the like}; bromine-based flame retardants {tetrabromophthalic anhydride, hexabromobenzene, decabromobiphenyl ether, and the like}; phosphoric acid ester-based flame retardants {tris(tribromophenyl)phosphate and the like}, and the like.

In the case where the curable composition contains a flame retardant, the content of the flame retardant is preferably 0.5 to 40000 parts by weight and further preferably 5 to 10000 parts by weight relative to 100 parts of the acid generating agent.

As the defoaming agent, a known defoaming agent and the like can be used, and examples thereof include alcoholic defoaming agents, metal soap-based defoaming agents, phosphoric acid ester-based defoaming agents, fatty acid ester-based defoaming agents, polyether-based defoaming agents, silicone-based defoaming agents, mineral oil-based defoaming agents, and the like.

As the fluidity regulating agent, a known fluidity regulating agent and the like can be used, and examples thereof include a hydrogenated castor oil, polyethyleneoxide, organic bentonite, colloidal silica, amide wax, metal soap, an acrylic ester polymer, and the like.

As the light stabilizer, a known light stabilizer and the like can be used, and examples thereof include ultraviolet ray-absorbing type stabilizers {benzotriazole, benzophenone, salicylate, and cyanoacrylate, derivatives thereof, and the like}; radical-scavenging type stabilizers {hindered amines and the like}; quenching type stabilizers {nickel complexes and the like}, and the like.

As the oxidation inhibitor, a known oxidation inhibitor and the like can be used, and examples thereof include phenol-based oxidation inhibitors (monophenol-based one, bisphenol-based one, phenol polymer-based one, and the like), sulfur-based oxidation inhibitors, phosphorus-based oxidation inhibitors, and the like.

As the adhesion imparting agent, a known adhesion imparting agent and the like can be used, and examples thereof include coupling agents, silane coupling agents, titanate coupling agents, and the like.

As the ion scavenger, a known ion scavenger and the like can be used, and examples thereof include respective kinds of organoaluminum (alkoxyaluminum, phenoxyaluminum, and the like), and the like.

As the coloration inhibitor, a known coloration inhibitor can be used, an oxidation inhibitor is generally effective, and examples thereof include phenol-based oxidation inhibitors (monophenol-based one, bisphenol-based one, phenol polymer-based one, and the like), sulfur-based oxidation inhibitors, phosphorus-based oxidation inhibitors, and the like.

In the case where the curable composition contains a defoaming agent, a fluidity regulating agent, a light stabilizer, an oxidation inhibitor, an adhesion imparting agent, an ion scavenger, or a coloration inhibitor, the content of each of those is preferably 0.1 to 20000 parts by weight and further preferably 0.5 to 5000 parts by weight relative to 100 parts of the acid generating agent.

No restriction is put on the solvent as long as the solvent can be used for the dissolution of a cationic polymerizable compound or the viscosity control of a curable composition, and as the solvent, those exemplified as solvents for the above-described acid generating agent can be used.

In the case where the curable composition contains a solvent, the content of the solvent is preferably 50 to 2000000 parts by weight and further preferably 200 to 500000 parts by weight relative to 100 parts of the acid generating agent.

Examples of the non-reactive resin include polyesters, polyvinyl acetate, polyvinyl chloride, polybutadiene, polycarbonates, polystyrenes, polyvinyl ethers, polyvinyl butyral, polybutenes, a hydrogenated product of a styrene/butadiene block copolymer, a copolymer of a (meth)acrylic acid ester, polyurethanes, and the like. The number average molecular weight of each of these resins is preferably 1000 to 500000 and further preferably 5000 to 100000 (the number average molecular weight is a measured value by a general method such as GPC or the like).

In the case where the curable composition contains a non-reactive resin, the content of the non-reactive resin is preferably 5 to 400000 parts by weight and further preferably 50 to 150000 parts by weight relative to 100 parts of the acid generating agent.

In the case of making the curable composition contain a non-reactive resin, it is desirable that the non-reactive resin be previously dissolved in a solvent in order to facilitate the compatibilization with a cationic polymerizable compound and the like.

As the radical polymerizable compound, a known {"Photopolymer Handbook" edited by Technical Association of Photopolymers, (1989, published by Kogyo Chosakai Publishing Co., Ltd.), "Technology of UV/EB Curing" edited by United Engineering Center, (1982, published by United Engineering Center), "UV/EB Curable Materials" edited by RadTech Japan, (1992, published by CMC Publishing Co., Ltd.), "UV koka ni okeru koka furyo•sogai genin to sono taisaku" edited by TECHNICAL INFORMATION INSTITUTE CO., LTD., (2003, published by TECHNICAL INFORMATION INSTITUTE CO., LTD.)} radical polymerizable compound and the like can be used, and monofunctional monomers, bifunctional monomers, polyfunctional monomers, epoxy (meth)acrylates, polyester (meth)acrylates, and urethane (meth)acrylates are included therein.

In the case where the curable composition contains a radical polymerizable compound, the content of the radical polymerizable compound is preferably 5 to 400000 parts by weight and further preferably 50 to 150000 parts by weight relative to 100 parts of the acid generating agent.

In the case where the curable composition contains a radical polymerizable compound, it is preferred that a radical polymerization initiator which is decomposed by heat or light to initiate polymerization be used in order to attain a heightened molecular weight by subjecting these compounds to radical polymerization.

As the radical polymerization initiator, a known radical polymerization initiator and the like can be used, and thermal radical polymerization initiators (organic peroxides, azo compounds, and the like) and photo-radical polymerization initiators (acetophenone-based initiators, benzophenone-based initiators, Michler's ketone-based initiators, benzoin-based initiators, thioxanthone-based initiators, acyl phosphine-based initiators, and the like) are included therein.

In the case where the curable composition contains a radical polymerization initiator, the content of the radical polymerization initiator is preferably 0.01 to 20 parts by weight and further preferably 0.1 to 10 parts by weight relative to 100 parts of the radical polymerizable compound.

The curable composition of the present invention can be prepared by uniformly mixing a cationic polymerizable compound, an acid generating agent, and an optional additive at room temperature (20 to 30°C or so) or under heating (40 to 90°C or so) as necessary to be formed into a liquid or further kneading the liquid with a three roll mill or the like.

The curable composition of the present invention can be cured by being heated or irradiated with an energy ray to obtain a cured product.

Any energy ray is acceptable as long as the energy ray has sufficient energy to induce the decomposition of a sulfonium borate salt of the present invention, and energy rays in ultraviolet to visible light regions (wavelength range: about 100 to about 800 nm) emitted from a low, medium, high, or ultra-high pressure mercury lamp, a metal halide lamp, an LED lamp, a xenon lamp, a carbon arc lamp, a fluorescent lamp, a light source of a semiconductor/solid laser, argon laser, He-Cd laser, KrF excimer laser, ArF excimer laser, or F₂ laser, and the like are preferred. In this connection, as the energy ray, a radioactive ray being high in energy density such as an electron beam or an X-ray can also be used.

Although the irradiation time of an energy ray is affected by the energy ray intensity and the energy ray permeability of a curable composition, a period of 0.1 seconds to 10 seconds or so is sufficient at ordinary temperature (20 to 30°C or so) . However, in the case where the energy ray permeability is low or in the case where the film thickness of a curable composition is thick, a period more than the above is sometimes taken preferably. Although most of the curable compositions are cured through cationic polymerization at the end of 0.1 seconds to several minutes after the energy ray irradiation, if necessary, after the irradiation with an energy ray, the composition can also be heated for several seconds to several hours at room temperature (20 to 30°C or so) to 150°C to be subjected to post-curing.

As a heating method by which a curable composition is cured, conventionally known methods such as a heat circulation type heating method, an infrared heating method, and a high frequency heating method can be used.

The heating temperature required for curing is not particularly limited as long as the heating temperature lies within a range where curing proceeds sufficiently and a base material is not degraded, and lies within the range of preferably 50°C to 250°C and more preferably 80°C to 200°C, and the heating time varies with the heating temperature, but the heating time is preferably several minutes to several hours from an aspect of productivity.

Specific examples of the application of a curable composition of the present invention include a paint, a coating agent, an ink, an inkjet ink, a positive type resist, a resist film, a liquid resist, a negative type resist, a resist for MEMS, a positive type photosensitive material, a negative type photosensitive material, various kinds of adhesives, a molding material, a casting material, putty, an impregnant for glass fiber, a filling material, a sealing material, an encapsulation material, an optical semiconductor (LED) encapsulation material, a material for an optical waveguide, a nanoimprint material, materials for photofabrication and for micro-photofabrication, and the like.

### EXAMPLES

Hereinafter, the present invention will be further described by reference to examples, but the present invention is not intended to be limited thereto. It should be noted that a part means a part by weight and % means % by weight unless otherwise stated.

### [Production Example 1] Synthesis of 4-(Ethylthio)phenol

In 20 ml of acetone, 5.00 g (0.04 mol) of 4-hydroxybenzenethiol and 6.18 g (0.04 mol) of iodoethane were dissolved and added with 6.02 g (0.44 mol) of potassium carbonate, and the mixture were allowed to undergo a reaction for 10 hours at 40°C. After the reaction, the reaction liquid was added with 20 ml of ethyl acetate and washed three times with 50-ml portions of ion-exchanged water, and the solvent was removed to obtain 4.84 g of 4-(ethylthio)phenol.

### [Production Example 2] Synthesis of 4-(Propylthio)phenol

In place of 6.18 g of iodoethane, 6.62 g of iodopropane was used to synthesize 4-(propylthio)phenol according to the method described in Production Example 1.

### [Production Example 3] Synthesis of 4-(Butylthio)phenol

In place of 6.18 g of iodoethane, 7.29 g of iodobutane was used to synthesize 4-(butylthio)phenol according to the method described in Production Example 1.

### [Example 1] Synthesis of Acid generating agent A-1

In 15 ml of methanol, 2.16 g (0.01 mol) of p-nitrobenzyl bromide and 1.40 g (0.01 mol) of 4-(methylthio)phenol were dissolved and stirred for 12 hours at 50°C. The solution was added with 30 ml of ion-exchanged water and 15 ml of ethyl acetate, stirred for 30 minutes, and then, separated into two layers of an ethyl acetate layer and a water layer to remove the ethyl acetate layer. With the water layer, 70 g of an aqueous solution containing an equimolar amount of sodium tetrakis(pentafluorophenyl)borate and 30 ml of ethyl acetate were mixed under room temperature and subsequently stirred for 3 hours. An ethyl acetate layer was washed three times with water by liquid separating operation, after which the solvent was distilled off by means of a rotary evaporator to obtain 8.22 g of a white solid. It was confirmed by 1H-NMR that this white solid was Acid generating agent A-1. The structure of A-1 was described in Table 1.

### [Example 2] Synthesis of Acid generating agent A-2

In place of 2.16 g of p-nitrobenzyl bromide, 2.16 g of m-nitrobenzyl bromide was used to prepare Acid generating agent A-2 according to the method described in Example 1. The structure of Acid generating agent A-2 was described in Table 1.

### [Example 3] Synthesis of Acid generating agent A-3

In place of 2.16 g of p-nitrobenzyl bromide, 1.96 g of p-cyanobenzyl bromide was used to prepare Acid generating agent A-3 according to the method described in Example 1. The structure of Acid generating agent A-3 was described in Table 1.

### [Example 4] Synthesis of Acid generating agent A-4

In place of 2.16 g of p-nitrobenzyl bromide, 1.96 g of m-cyanobenzyl bromide was used to prepare Acid generating agent A-4 according to the method described in Example 1. The structure of Acid generating agent A-4 was described in Table 1.

### [Example 5] Synthesis of Acid generating agent A-5

In place of 2.16 g of p-nitrobenzyl bromide, 2.39 g of p-trifluoromethylbenzyl bromide was used to prepare Acid generating agent A-5 according to the method described in Example 1. The structure of Acid generating agent A-5 was described in Table 1.

### [Example 6] Synthesis of Acid generating agent A-6

In place of 2.16 g of p-nitrobenzyl bromide, 2.39 g of m-trifluoromethylbenzyl bromide was used to prepare Acid generating agent A-6 according to the method described in Example 1. The structure of Acid generating agent A-6 was described in Table 1.

### [Example 7] Synthesis of Acid generating agent A-7

In place of 2.16 g of p-nitrobenzyl bromide, 2.61 g of pentafluorobenzyl bromide was used to prepare Acid generating agent A-7 according to the method described in Example 1. The structure of Acid generating agent A-7 was described in Table 1.

### [Example 8] Synthesis of Acid generating agent A-8

In place of 2.16 g of p-nitrobenzyl bromide, 3.07 g of 3,5-trifluoromethylbenzyl bromide was used to prepare Acid generating agent A-8 according to the method described in Example 1. The structure of Acid generating agent A-8 was described in Table 1.

### [Example 9] Synthesis of Acid generating agent A-9

In place of 2.16 g of p-nitrobenzyl bromide, 3.07 g of 2,4-trifluoromethylbenzyl bromide was used to prepare Acid generating agent A-9 according to the method described in Example 1. The structure of Acid generating agent A-9 was described in Table 1.

### [Example 10] Synthesis of Acid generating agent A-10

In place of 2.16 g of p-nitrobenzyl bromide, 3.07 g of 2,5-trifluoromethylbenzyl bromide was used to prepare Acid generating agent A-10 according to the method described in Example 1. The structure of Acid generating agent A-10 was described in Table 1.

### [Example 11] Synthesis of Acid generating agent A-11

In place of 1.40 g of 4-(methylthio)phenol, 1.54 g of 4-(ethylthio)phenol synthesized in Production Example 1 was used to prepare Acid generating agent A-11 according to the method described in Example 1. The structure of Acid generating agent A-11 was described in Table 1.

### [Example 12] Synthesis of Acid generating agent A-12

In place of 1.40 g of 4-(methylthio)phenol, 1.65 g of 4-(propylthio)phenol synthesized in Production Example 2 was used to prepare Acid generating agent A-12 according to the method described in Example 1. The structure of Acid generating agent A-12 was described in Table 1.

### [Example 13] Synthesis of Acid generating agent A-13

In place of 1.40 g of 4-(methylthio)phenol, 1.82 g of 4-(butylthio)phenol synthesized in Production Example 3 was used to prepare Acid generating agent A-13 according to the method described in Example 1. The structure of Acid generating agent A-13 was described in Table 1.

### [Comparative Example 1] Synthesis of Acid generating agent H-1

In place of 2.16 g of p-nitrobenzyl bromide, 1.27 g of benzyl chloride was used to prepare Acid generating agent H-1 according to the method described in Example 1. The structure of Acid generating agent H-1 was described in Table 1.

### [Comparative Example 2] Synthesis of Acid generating agent H-2

In place of 2.16 g of p-nitrobenzyl bromide, 1.45 g of p-fluorobenzyl chloride was used to prepare Acid generating agent H-2 according to the method described in Example 1. The structure of Acid generating agent H-2 was described in Table 1.

### [Comparative Example 3] Synthesis of Acid generating agent H-3

In place of 2.16 g of p-nitrobenzyl bromide, 1.61 g of p-chlorobenzyl chloride was used to prepare Acid generating agent H-3 according to the method described in Example 1. The structure of Acid generating agent H-3 was described in Table 1.

### [Comparative Example 4] Synthesis of Acid generating agent H-4

In place of 2.16 g of p-nitrobenzyl bromide, 2.50 g of p-bromobenzyl bromide was used to prepare Acid generating agent H-4 according to the method described in Example 1. The structure of Acid generating agent H-4 was described in Table 1.

### [Comparative Example 5] Synthesis of 4-Hydroxyphenyldimethylsulfonium tetrakis(pentafluorophenyl)borate (H-5)

In 15 ml of dichloromethane, 1.92 g (0.01 mol) of 4-hydroxyphenyldimethylsulfonium chloride was dispersed, and with this dispersion, 70 g of an aqueous solution containing an equimolar amount of sodium tetrakis(pentafluorophenyl)borate was mixed under room temperature and subsequently stirred for 3 hours. A dichloromethane layer was washed three times with water by liquid separating operation and then transferred to a rotary evaporator, and the solvent was distilled off to obtain 4-hydroxyphenyldimethylsulfonium tetrakis(pentafluorophenyl)borate.

### [Comparative Example 6] Synthesis of 4-Acetoxyphenyldimethylsulfonium tetrakis(pentafluorophenyl)borate (H-6)

In 100 ml of acetonitrile, 6.00 g (0.01 mol) of 4-hydroxyphenyldimethylsulfonium tetrakis(pentafluorophenyl)borate synthesized in Comparative Example 5 was dissolved, and to this solution, 1.22 g (0.012 mol) of triethylamine was added under the condition of 10°C or less, and at the end of 30 minutes after the addition, 0.95 g (0.012 mol) of acetyl chloride was added dropwise. After being stirred for 3 hours, triethylamine hydrochloride produced as a by-product was filtered to be removed, the filtrate was transferred to a rotary evaporator, and the solvent was distilled off to obtain 4-acetoxyphenyldimethylsulfonium tetrakis(pentafluorophenyl)borate.

**[Table 1]**

| | R1 | R3 | R4 | R5 | R6 | R7 | Sum of Hammett substituent constant σ values of R3 to R7 |
|---|---|---|---|---|---|---|---|
| A-1 | CH₃ | H | H | NO₂ | H | H | 0.78 |
| A-2 | CH₃ | H | NO₂ | H | H | H | 0.71 |
| A-3 | CH₃ | H | H | CN | H | H | 0.66 |
| A-4 | CH₃ | H | CN | H | H | H | 0.56 |
| A-5 | CH₃ | H | H | CF₃ | H | H | 0.54 |
| A-6 | CH₃ | H | CF₃ | H | H | H | 0.43 |
| A-7 | CH₃ | F | F | F | F | F | 0.86 |
| A-8 | CH₃ | H | CF₃ | H | CF₃ | H | 0.86 |
| A-9 | CH₃ | CF₃ | H | CF₃ | H | H | 1.08 |
| A-10 | CH₃ | CF₃ | H | H | CF₃ | H | 0.97 |
| A-11 | C₂H₅ | H | H | NO₂ | H | H | 0.78 |
| A-12 | C₃H₇ | H | H | NO₂ | H | H | 0.78 |
| A-13 | C₄H₉ | H | H | NO₂ | H | H | 0.78 |
| H-1 | CH₃ | H | H | H | H | H | 0 |
| H-2 | CH₃ | H | H | F | H | H | 0.06 |
| H-3 | CH₃ | H | H | cl | H | H | 0.22 |
| H-4 | CH₃ | H | H | Br | H | H | 0.23 |

### (Preparation of Heat-curable composition and Evaluation thereof)

An acid generating agent of the present invention or a compound of the comparative example in a blending amount shown in Table 2 was dissolved in propylene carbonate, and then, uniformly mixed with an epoxide (a bisphenol A type epoxy resin, available from Mitsubishi Chemical Corporation, Epikote 828) being a cationic polymerizable compound in a blending amount (parts by weight) of Table 2 to prepare each of heat-curable compositions (Examples C1 to C10, Comparative Examples C1 to C6) .

**[Table 2]**

| | Acid generating agent | Blending amount of Acid generating agent | Propylene carbonate | Epoxide |
|---|---|---|---|---|
| Example C1 | A-1 | 0.1 | 0.1 | 100 |
| Example C2 | A-2 | 0.1 | 0.1 | 100 |
| Example C3 | A-3 | 0.1 | 0.1 | 100 |
| Example C4 | A-4 | 0.1 | 0.1 | 100 |
| Example C5 | A-5 | 0.1 | 0.1 | 100 |
| Example C6 | A-6 | 0.1 | 0.1 | 100 |
| Example C7 | A-7 | 0.1 | 0.1 | 100 |
| Example C8 | A-8 | 0.1 | 0.1 | 100 |
| Example C9 | A-9 | 0.1 | 0.1 | 100 |
| Example C10 | A-10 | 0.1 | 0.1 | 100 |
| Example C11 | A-11 | 0.1 | 0.1 | 100 |
| Example C12 | A-12 | 0.1 | 0.1 | 100 |
| Example C13 | A-13 | 0.1 | 0.1 | 100 |
| Comparative Example C1 | H-1 | 0.1 | 0.1 | 100 |
| Comparative Example C2 | H-2 | 0.1 | 0.1 | 100 |
| Comparative Example C3 | H-3 | 0.1 | 0.1 | 100 |
| Comparative Example C4 | H-4 | 0.1 | 0.1 | 100 |
| Comparative Example C5 | H-5 | 0.1 | 0.1 | 100 |
| Comparative Example C6 | H-6 | 0.1 | 0.1 | 100 |

### <Gel time (Thermosetting properties)>

The heat-curable compositions obtained as above were measured for the gel time in accordance with the procedure of JISK5909.

These results were shown in Table 3.

### <Storage stability>

The heat-curable compositions obtained as above were preserved for 1 week at 40°C under a light-shielded condition, after which formulation samples thereof were measured for the viscosity to be evaluated according to the following criteria. These results were shown in Table 3.

### (Evaluation criteria)

×: The change in viscosity after 1 week is 2.0 times or more.
○: The change in viscosity after 1 week is less than 2.0 times.
⊙: The change in viscosity after 2 weeks is less than 2.0 times.

**[Table 3]**

| | Gel time | Storage stability |
|---|---|---|
| | | |
| Example C1 | Three minutes and twenty seconds | ⊙ |
| Example C2 | Three minutes and twenty nine seconds | ⊙ |
| Example C3 | Three minutes and nine seconds | ○ |
| Example C4 | Three minutes and two seconds | ○ |
| Example C5 | Two minutes and fifty eight seconds | ○ |
| Example C6 | Two minutes and fifty three seconds | ○ |
| Example C7 | Three minutes and thirty seconds | ⊙ |
| Example C8 | Three minutes and forty three seconds | ⊙ |
| Example C9 | Three minutes and fifty eight seconds | ⊙ |
| Example C10 | Three minutes and forty eight seconds | ⊙ |
| Example C11 | Three minutes and zero seconds | ⊙ |
| Example C12 | Two minutes and fifty nine seconds | ⊙ |
| Example C13 | Two minutes and fifty three seconds | ○ |
| Comparative Example C1 | Two minutes and thirty seconds | × |
| Comparative Example C2 | Two minutes and twenty seven seconds | × |
| Comparative Example C3 | Two minutes and thirty three seconds | × |
| Comparative Example C4 | Two minutes and fifty three seconds | × |
| Comparative Example C5 | Longer than thirty minutes | ○ |
| Comparative Example C6 | Longer than thirty minutes | ○ |

The results in Table 3 revealed that the sulfonium borate salt of the present invention was excellent in balance between thermosetting properties and storage stability compared with sulfonium borate salts for comparison.

### INDUSTRIAL APPLICABILITY

The sulfonium borate salt of the present invention is suitably used as an acid generating agent used for a paint, a coating agent, an ink, an inkjet ink, a positive type resist (a connecting terminal, wiring pattern formation, or the like in the production of electronic components such as a circuit board, CSP, and an MEMS element), a resist film, a liquid resist, a negative type resist (a permanent film material such as a surface protective film, an interlayer insulating film, and a flattening film for a semiconductor element or the like, and the like), a resist for MEMS, a photosensitive material, various kinds of adhesives, a molding material, a casting material, putty, an impregnant for glass fiber, a filling material, a sealing material, an encapsulation material, an optical semiconductor (LED) encapsulation material, a nanoimprint material, materials for photofabrication and for micro-photofabrication, and the like.

## Claims

1. A sulfonium borate salt represented by the following general formula (1). 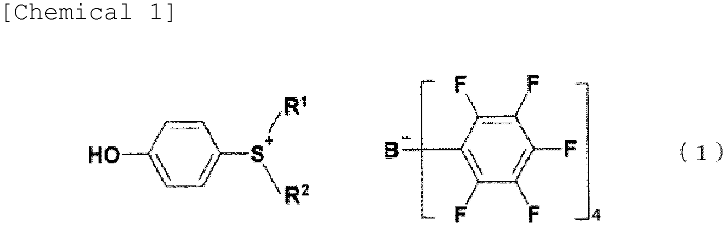 [In formula (1), R¹ represents an alkyl group, and R² represents a benzyl group represented by the following general formula (2).] 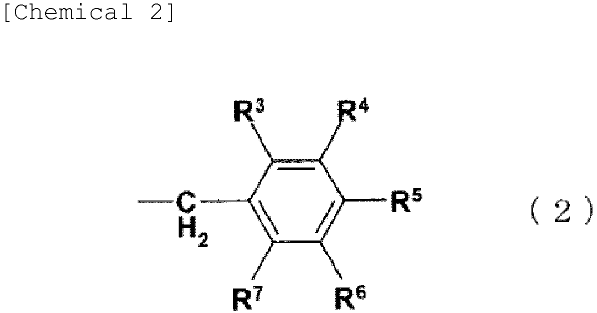 [In formula (2), R³ to R⁷ independently represent a hydrogen atom or an electron-attracting group, and the sum of Hammett substituent constant σ values of R³ to R⁷ is 0.43 or more.]

2. The sulfonium borate salt according to claim 1, wherein R² in formula (1) represents any one of a p-nitrobenzyl group, a m-nitrobenzyl group, a p-cyanobenzyl group, a m-cyanobenzyl group, a p-trifluoromethylbenzyl group, a m-trifluoromethylbenzyl group, a pentafluorobenzyl group, a 3,5-bis(trifluoromethyl)benzyl group, a 2,4-bis(trifluoromethyl)benzyl group, and a 2,5-bis(trifluoromethyl)benzyl group.

3. An acid generating agent, comprising the sulfonium borate salt according to claim 1 or 2.

4. A curable composition, including the acid generating agent according to claim 3 and a cationic polymerizable compound.

5. A cured product obtained by curing the curable composition according to claim 4.
